# FASCICULE DE BREVET EUROPEEN

(11) **EP 3 659 557 B1**
(45) Date de publication et mention de la délivrance du brevet: **20.12.2023**
(21) Numéro de dépôt: 18208586.0
(22) Date de dépôt: 27.11.2018
(51) Int. Cl.: A61F 2/46, A61F 2/42

(54) **PROCÉDÉ DE FABRICATION D'UN DISPOSITIF D'AIDE A LA POSE D'UNE PROTHESE TRAPEZO-METACARPIENNE**
VERFAHREN ZUR HERSTELLUNG EINER HILFSVORRICHTUNG FÜR DAS EINSETZEN EINER TRAPEZIOMETACARPALEN PROTHESE
METHOD OF MANUFACTURING A DEVICE FOR AIDING IN THE FITTING OF A TRAPEZIOMETACARPAL PROSTHESIS

(43) Date de publication de la demande: 03.06.2020
(73) Titulaire: Keri Medical SA, 1227 Les Acacias (CH)
(72) Inventeur: Prandi, Bernard, 6006 Lucerne (CH); Altheer, Christian, 1217 Meyrin (CH); Mottet, Julie, 74930 Pers-Jussy (FR); Maurice, Eric, 33200 Bordeaux (FR); Duerinckx, Joris, 3600 Genk (BE)
(74) Mandataire: Micheli & Cie SA

(56) Documents cités:
- EP-A1- 2 564 802
- WO-A1-97/42895
- WO-A1-2014/206498
- US-A1- 2009 254 190

## Description

La présente invention concerne un procédé de fabrication d'un dispositif d'aide à la pose d'une prothèse trapézo-métacarpienne.

Une prothèse trapézo-métacarpienne est indiquée pour des patients souffrant de rhizarthose et permet de remplacer l'articulation de la base du pouce par une articulation artificielle. Elle est composée essentiellement de deux parties, à savoir une cupule destinée à être fixée dans le trapèze du patient et une tige destinée à être fixée dans le premier métacarpien du patient. Un insert est monté à l'extrémité de la tige, généralement au moyen d'un col, et coopère avec la cupule pour reproduire l'articulation trapézo-métacarpienne. Un exemple de prothèse trapézo-métacarpienne est décrit dans le document de brevet US 2009/0254190, qui décrit un dispositif permettant au chirurgien de choisir une taille appropriée pour l'implant trapézien.

Contrairement aux prothèses prévues pour le genou, une prothèse trapézo-métacarpienne est difficile à mettre en place. En effet, lors d'une opération au niveau d'un genou, l'ensemble de l'articulation entre le fémur et le tibia est parfaitement visible et accessible, de sorte qu'il est facile de positionner les différents guides et instruments nécessaires à la pose d'une prothèse de genou. Au contraire, l'articulation entre le pouce et le premier métacarpien est difficilement accessible, seul un très faible écartement entre le pouce et le premier métacarpien étant possible. En outre, lors d'une opération au niveau de la base du pouce, l'incision pratiquée est la plus petite possible afin d'être peu invasive. Il est de ce fait difficile pour le chirurgien de trouver le bon angle pour positionner ses différents guides ou outils, tels qu'une broche de guidage. De plus, la surface articulaire distale du trapèze présente une forme en selle, concave dans le plan frontal et convexe dans le plan sagittal, de sorte qu'il est difficile pour le chirurgien de trouver une surface plane pour pouvoir positionner correctement la cupule.

Certains guides de coupe ont été développés pour aider à positionner une scie utilisée pour la résection du premier métacarpien. De tels guides de coupe se positionnent sur le premier métacarpien avant sa résection puis sont ensuite retirés de sorte que les éventuels repères qui auraient pu être pris par rapport à la configuration initiale de l'anatomie du patient sont perdus.

En conséquence, le chirurgien doit essentiellement opérer « à vue » et doit effectuer régulièrement des radios de contrôle afin de vérifier le bon positionnement de ses différents guides et outils au cours de l'intervention. De plus, l'articulation trapézo-métacarpienne est propre à chaque patient, de sorte que le chirurgien doit systématiquement adapter ses gestes à l'anatomie du patient. La pose d'une prothèse trapézo-métacarpienne est donc une opération délicate qui nécessite beaucoup de pratique pour pouvoir être parfaitement réalisée.

La présente invention vise à proposer un procédé de fabrication d'un dispositif d'aide à la pose d'une prothèse trapézo-métacarpienne qui permette au chirurgien de positionner ladite prothèse d'une manière optimale en fonction de l'anatomie de son patient.

A cet effet, la présente invention concerne un procédé de fabrication d'un dispositif d'aide à la pose d'une prothèse trapézo-métacarpienne comprenant une cupule destinée à être fixée dans le trapèze d'un patient et une tige destinée à être fixée dans le premier métacarpien du patient.

Selon l'invention, on réalise ledit dispositif de telle sorte qu'il comprend un premier guide apte à être interposé entre le trapèze et le premier métacarpien du patient, ledit premier guide comprenant un premier corps présentant une première surface destinée à être positionnée sur la surface articulaire du trapèze, ladite première surface étant congruente à ladite surface articulaire du trapèze, et une deuxième surface destinée à être en regard de la surface articulaire du premier métacarpien avant résection, le premier guide comprenant également des organes de repérage portés par le premier corps et agencés pour définir des lignes de résection du premier métacarpien. En outre, selon l'invention, on réalise le premier guide au moins partiellement sur mesure à l'aide d'une imagerie médicale en trois dimensions au moins du trapèze et du premier métacarpien du patient, de manière préopératoire.

Un tel dispositif d'aide à la pose d'une prothèse trapézo-métacarpienne permet un positionnement sûr et précis du premier guide afin de repérer de manière optimale les lignes de résection du premier métacarpien.

D'une manière particulièrement avantageuse, on peut réaliser ledit dispositif d'aide de telle sorte qu'il comprend un second guide agencé pour être positionné sur le premier guide après résection du premier métacarpien, ledit second guide comprenant un second corps présentant une troisième surface destinée à reposer sur la deuxième surface du premier guide, et une quatrième surface destinée à être en regard du premier métacarpien réséqué, ladite troisième surface étant congruente avec la deuxième surface du premier guide, ledit second corps du second guide comprenant au moins sur sa quatrième surface au moins un orifice de guidage agencé pour recevoir un outil.

De préférence, on réalise ledit dispositif de telle sorte que le premier corps du premier guide comprend également au moins sur sa deuxième surface au moins un orifice de guidage agencé pour recevoir un outil, les orifices de guidage étant alignés, traversants et communicants.

Un tel dispositif d'aide à la pose d'une prothèse trapézo-métacarpienne permet un positionnement sûr et précis d'un outil, tel qu'une broche de guidage, nécessaire à la mise en place de la prothèse.

De préférence, le second guide est réalisé de manière préopératoire spécifiquement pour le patient par imagerie 3D.

D'autres caractéristiques et avantages de la présente invention apparaîtront à la lecture de la description détaillée suivante faite en référence aux dessins annexés dans lesquels :
- la figure 1 est une vue en perspective d'un premier guide d'un dispositif d'aide fabriqué selon le procédé selon l'invention, interposé entre le trapèze et le premier métacarpien d'un patient;
- la figure 2 est une vue éclatée de la figure 1 ;
- la figure 3 est une vue de dessus d'un premier guide d'un dispositif d'aide fabriqué selon le procédé selon l'invention, interposé entre le trapèze et le premier métacarpien d'un patient ;
- la figure 4 est une vue de face du trapèze portant le premier guide d'un dispositif d'aide fabriqué selon le procédé selon l'invention ;
- la figure 5 est une vue de face d'un canon de perçage utilisé avec le premier guide représenté sur la figure 4 ; et
- la figure 6 est une vue en perspective d'un premier guide et d'un second guide d'un dispositif d'aide fabriqué selon le procédé selon l'invention, positionnés sur le trapèze et traversés par une broche de guidage.

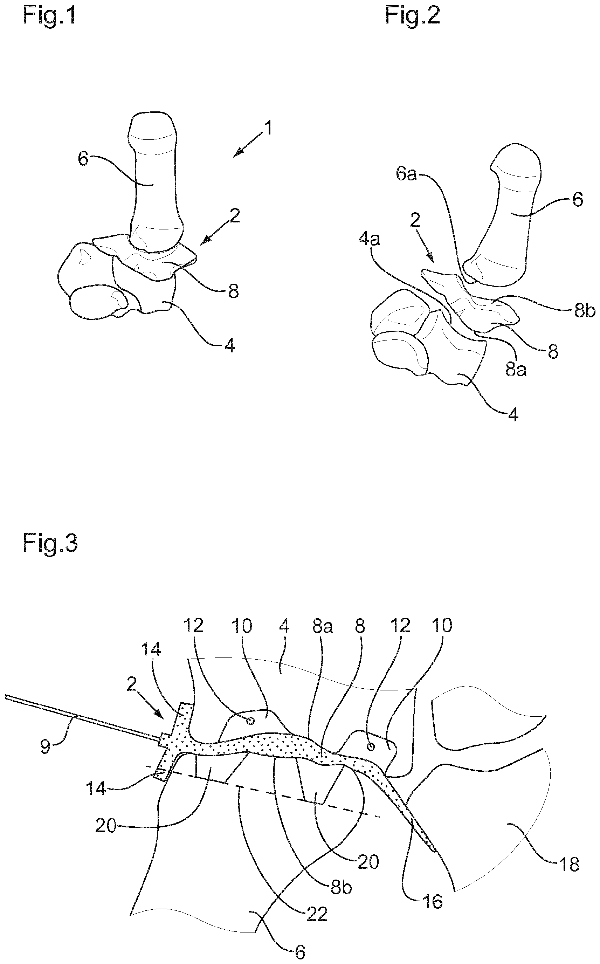

En référence aux figures 1 à 3, le dispositif 1 d'aide à la pose d'une prothèse trapézo-métacarpienne fabriqué selon le procédé selon l'invention comprend un premier guide 2 apte à être interposé entre le trapèze 4 et le premier métacarpien 6 d'un patient. Ledit premier guide 2 comprend un premier corps 8 présentant une première surface transversale 8a destinée à être positionnée sur la surface articulaire distale 4a en forme de selle du trapèze 4, et plus spécifiquement destinée à être positionnée au contact de ladite surface articulaire distale 4a. A cet effet, ladite première surface 8a est congruente à la surface articulaire distale 4a du trapèze 4 de sorte que les deux surfaces 8a et 4a s'adaptent et s'imbriquent parfaitement l'une à l'autre. A l'opposé de la première surface 8a par rapport à un plan transversal, le premier corps 8 du premier guide 2 présente une deuxième surface transversale 8b destinée à venir en regard de la surface articulaire proximale 6a du premier métacarpien 6, avant résection. D'une manière particulièrement préférée, la deuxième surface 8b est apte à être positionnée au contact de la surface articulaire proximale 6a. A cet effet, et d'une manière préférentielle, ladite deuxième surface 8b est congruente à la surface articulaire proximale 6a du premier métacarpien 6 de sorte que les deux surfaces 8b et 6a s'adaptent et s'imbriquent parfaitement l'une à l'autre.

Les deux surfaces 8a et 8b du premier corps 8 du premier guide 2 étant congruentes aux surfaces articulaires du trapèze 4 et du premier métacarpien 6 respectivement, il est possible d'interposer le premier guide 2 entre le trapèze 4 et le premier métacarpien 6 de manière stable et précise, et cela même si les surfaces articulaires 4a, 6a restant disponibles sont réduites, selon l'anatomie ou la pathologie du patient. La congruence des deux surfaces opposées 8a et 8b du premier corps 8 du premier guide 2 permet également un gain de place et ainsi de laisser un accès à d'autres outils ou instruments nécessaires à l'intervention.

Le premier guide 2 a été réalisé au moins partiellement, sur mesure à l'aide d'une imagerie médicale en trois dimensions (3D) au moins du trapèze 4 et du premier métacarpien 6 du patient, de manière préopératoire, comme cela sera détaillé ci-après. Notamment, la configuration des surfaces 8a et 8b a été définie à partir d'un modèle virtuel 3D spécifique au patient des surfaces articulaires 4a, 6a du trapèze 4 et du premier métacarpien 6 respectivement afin que lesdites surfaces 8a et 8b du premier corps 8 soient parfaitement congruentes auxdites surfaces articulaires 4a et 6a respectivement.

Un manche 9 peut être prévu à l'extrémité dirigée vers l'extérieur du premier guide 2 pour faciliter sa manipulation.

Bien que la stabilité du premier guide 2 entre le trapèze 4 et le premier métacarpien 6 soit déjà assurée par ses deux surfaces transversales congruentes 8a, 8b, il est possible de prévoir sur le premier guide 2 des premiers organes de fixation agencés pour venir se fixer sur des os adjacents, notamment sur le trapèze 4, pour renforcer au moins temporairement le maintien du premier corps 8 du premier guide 2 audit trapèze 4, en particulier lorsque les surfaces congruentes 8a, 8b sont réduites. Ces premiers organes de fixation peuvent avantageusement consister en des pattes de fixation 10 ou des ergots faisant saillie du premier corps 8 au-dessus de la face dorsale du trapèze 4 et agencées pour être fixées au trapèze 4 (ou autre os adjacent) par exemple par des vis 12 vissées dans des trous correspondants prévus à cet effet sur le trapèze 4, ou par tout autre moyen de fixation temporaire approprié.

De même, bien que le positionnement précis du premier guide 2 entre le trapèze 4 et le premier métacarpien 6 soit déjà assuré par ses deux surfaces congruentes 8a, 8b, il est possible de prévoir sur le premier guide 2 des organes de positionnement agencés pour garantir une interposition exacte du premier corps 8 du premier guide 2 entre le trapèze 4 et le premier métacarpien 6, en particulier lorsque les surfaces congruentes 8a, 8b sont réduites. Ces organes de positionnement peuvent avantageusement comprendre une ou plusieurs pattes de centrage 14 prévues par exemple à l'extrémité dirigée vers l'extérieur du premier guide 2, entre le manche 9 et le premier guide 2, et disposées sensiblement perpendiculairement à l'axe du manche 9, de part et d'autre dudit manche 9. Les pattes de centrage 14 sont positionnées de manière à venir en butée contre les faces latérales extérieures du trapèze 4 et du premier métacarpien 6 lorsque le premier corps 8 est correctement interposé entre le trapèze 4 et le premier métacarpien 6.

Les organes de positionnement peuvent également comprendre une ou plusieurs pattes de positionnement 16 prévues par exemple à l'extrémité dirigée vers l'intérieur du premier guide 2, disposées sensiblement dans le prolongement du premier corps 8 et configurées de manière à venir en butée contre la face inférieure du trapézoïde 18 lorsque le premier corps 8 est correctement interposé entre le trapèze 4 et le premier métacarpien 6.

Le positionnement exact des pattes de fixation 10, des pattes de centrage 14 et des pattes de positionnement 16 sur le premier guide 2 est déterminé spécifiquement pour le patient, par imagerie médicale 3D, de manière préopératoire, comme cela sera détaillé ci-après.

En outre, le premier guide 2 comprend des organes de repérage portés par le premier corps 8 et agencés pour définir des lignes de résection 22 du premier métacarpien 6. Ces organes de repérage peuvent être intégrés au premier guide 2 ou être amovibles, afin de pouvoir être retirés après la résection du premier métacarpien. Plus particulièrement, lesdits organes de repérage peuvent être des pattes de découpe 20, ou des ergots, formant une seule pièce avec le premier corps 8 ou étant amovibles, faisant saillie du premier corps 8 du premier guide 2 au-dessus de la face dorsale du premier métacarpien 6 et définissant par projection au-dessus de ladite face dorsale du premier métacarpien 6 les lignes de résection 22 dans un plan sensiblement perpendiculaire à l'axe du premier métacarpien 6.

Le positionnement exact des pattes de découpe 20 sur le premier guide 2 est déterminé spécifiquement pour le patient, par imagerie médicale 3D, de manière préopératoire, comme cela sera détaillé ci-après.

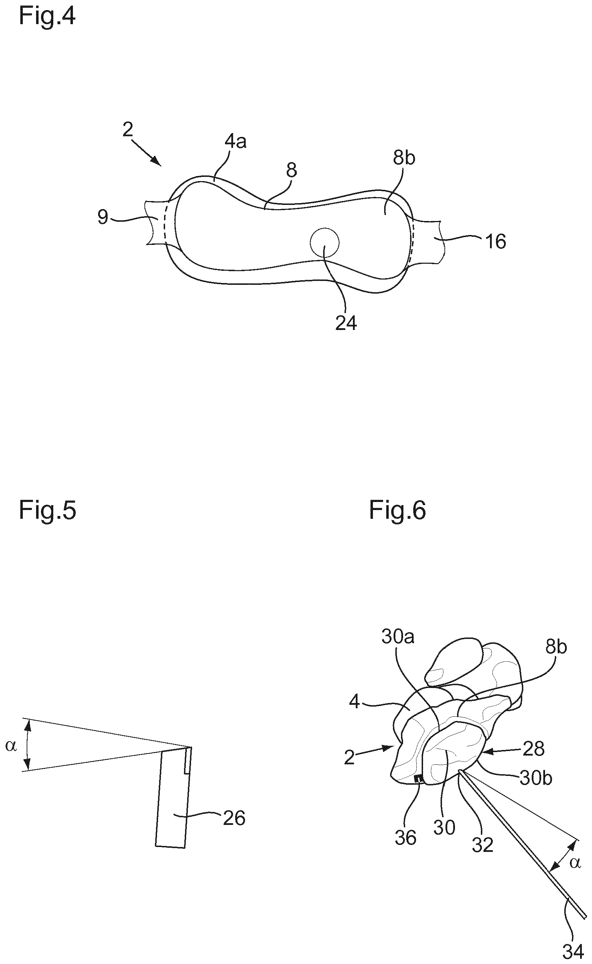

En outre, et en référence à la figure 4, le premier corps 8 du premier guide 2 peut comprendre au moins sur sa deuxième surface transversale 8b au moins un premier orifice de guidage 24 agencé pour recevoir un outil, après la résection de la base du premier métacarpien. Ce premier orifice de guidage 24 est positionné de manière préopératoire et permet au chirurgien au moins de repérer un point d'entrée pour positionner son outil, tel qu'une broche de guidage, pour préparer le trapèze.

D'une manière particulièrement préférée, le premier orifice de guidage 24 est un trou traversant débouchant dans la première surface transversale 8a pour pouvoir atteindre la surface articulaire 4a de la face inférieure du trapèze 4. L'angle d'inclinaison α du premier orifice de guidage 24 par rapport à un axe sensiblement perpendiculaire à la surface articulaire 4a du trapèze 4 est déterminé de manière préopératoire et permet au chirurgien d'orienter son outil, tel qu'une broche de guidage, positionné dans le premier orifice de guidage 24, selon le bon angle d'inclinaison.

Avantageusement, la position ainsi que l'angle d'inclinaison α du premier orifice de guidage 24 du premier guide ont été définis de manière préopératoire afin de permettre la mise en place d'un outil, tel qu'une broche de guidage, dans le premier guide 2 selon le meilleur positionnement et la meilleure orientation possibles par rapport à l'anatomie du trapèze 4 et du premier métacarpien 6 du patient.

De préférence, le positionnement et l'angle d'inclinaison exacts du premier orifice de guidage 24 sont déterminés spécifiquement pour le patient, par imagerie médicale 3D, de manière préopératoire, comme cela sera détaillé ci-après.

Afin d'améliorer le guidage d'un outil positionné dans le premier orifice de guidage 24 après la résection du premier métacarpien, il peut être prévu un canon de perçage 26, tel que représenté sur la figure 5, agencé pour être introduit dans le premier orifice de guidage 24. Le canon de perçage 26 présente une extrémité biseautée selon l'angle d'inclinaison α afin de respecter l'angle d'inclinaison déterminé pour le premier orifice de guidage 24. Par sécurité, un détrompeur correspondant au canon de perçage 26 peut être prévu dans le premier orifice de guidage 24.

L'adjonction du canon de perçage 26 sur le premier guide 2 après la résection de la base du premier métacarpien permet d'améliorer le positionnement et l'angle d'inclinaison d'un outil utilisé pour préparer le trapèze, tel qu'une broche de guidage sur laquelle sera introduite une fraise, telle qu'une fraise canulée.

Selon un mode de réalisation particulièrement préféré, et en référence à la figure 6, le dispositif d'aide selon la présente invention comprend en outre un second guide 28 utilisé à la place d'un canon de perçage 26. Le second guide 28 est agencé pour être positionné sur le premier guide 2 après résection de la base du premier métacarpien 6, et plus spécifiquement au contact de la deuxième surface transversale 8b du premier guide 2. A cet effet, ledit second guide 28 comprend un second corps 30 présentant une troisième surface transversale 30a destinée à reposer sur la deuxième surface transversale 8b du premier corps 8 du premier guide 2, ladite troisième surface 30a étant congruente avec la deuxième surface 8b du premier guide 2 de sorte que les deux surfaces 8b et 30a s'adaptent et s'imbriquent parfaitement l'une à l'autre. En d'autres termes, la troisième surface 30a du second guide 28 est identique à la surface articulaire 6a à la base du premier métacarpien 6. A l'opposé de la troisième surface transversale 30a par rapport à un plan transversal, le second corps 30 du second guide 28 présente une quatrième surface transversale 30b destinée à être en regard du premier métacarpien 6 réséqué.

La surface 8b du premier corps 8 du premier guide 2 et la surface 30a du second corps 30 du second guide 28 étant congruentes, il est possible de positionner le second guide 28 sur le premier guide 2 de manière stable et précise, et cela même si lesdites surfaces 8b et 30 sont réduites, selon l'anatomie ou la pathologie du patient. La congruence de la surface 8b du premier corps 8 du premier guide 2 et de la surface 30a du second corps 30 du second guide 28 permet également un gain de place.

Le second guide 28 a été réalisé au moins partiellement sur mesure à l'aide d'une imagerie médicale en trois dimensions (3D) au moins du trapèze et du premier métacarpien du patient, de manière préopératoire, comme cela sera détaillé ci-après. Notamment, la configuration de la surface 30a a été définie à partir d'un modèle virtuel 3D spécifique au patient de la surface articulaire 6a du premier métacarpien 6 afin que ladite surface 30a du second corps 30 soit parfaitement congruente à ladite surface articulaire 6a et par conséquent à la deuxième surface 8b du premier guide 2.

De plus, le second corps 30 du second guide 28 comprend au moins sur sa quatrième surface transversale 30b au moins un second orifice de guidage 32 agencé pour recevoir un outil, après la résection de la base du premier métacarpien 6. Ce second orifice de guidage 32 est positionné de manière préopératoire et permet au chirurgien au moins de repérer un point d'entrée pour positionner son outil, tel qu'une broche de guidage 34, pour préparer le trapèze.

D'une manière particulièrement préférée, le second orifice de guidage 32 est un trou traversant et est disposé de sorte que le premier orifice de guidage 24 et le second orifice de guidage 32 sont traversants, alignés et communicants. Ainsi, le second orifice de guidage 32 traverse le second guide 28 pour déboucher dans la deuxième surface transversale 8b de sorte que la surface articulaire 4a de la face inférieure du trapèze 4 peut ensuite être atteinte en traversant le premier orifice de guidage 24.

La quatrième surface transversale 30b est configurée pour que le second guide 28 présente une hauteur suffisante pour permettre un guidage sûr d'un outil tout en lui permettant d'être inséré entre le premier guide 2 et le premier métacarpien réséqué 6.

L'angle d'inclinaison α du second orifice de guidage 32 par rapport à un axe sensiblement perpendiculaire à la surface articulaire 4a du trapèze 4 est le même que défini précédemment. Il est déterminé de manière préopératoire et permet au chirurgien d'orienter son outil, tel qu'une broche de guidage 34, positionné dans le second orifice de guidage 32, selon le bon angle d'inclinaison.

Avantageusement, la position ainsi que l'angle d'inclinaison α du second orifice de guidage 32 du second guide 28 ont été définis de manière préopératoire afin de permettre la mise en place d'un outil, tel qu'une broche de guidage 34, dans le second guide 28 selon le meilleur positionnement et la meilleure orientation possibles par rapport à l'anatomie du trapèze 4 et du premier métacarpien 6 du patient.

De préférence, le positionnement et l'angle d'inclinaison exacts du second orifice de guidage 28 sont déterminés spécifiquement pour le patient, par imagerie médicale 3D, de manière préopératoire, comme cela sera détaillé ci-après.

Bien que la stabilité du second guide 28 sur le premier guide 2 soit déjà assurée par leurs surfaces congruentes 30a et 8b respectives, il est possible de prévoir sur le second guide 28 des seconds organes de fixation 36, tels que des ergots de fixation, agencés pour maintenir au moins temporairement le second corps 30 du second guide 28 sur le premier guide 2, en particulier lorsque les surfaces congruentes 30a, 8b sont réduites. Le positionnement exact des seconds organes de fixation 36 sur le second guide 28 est déterminé spécifiquement pour le patient, par imagerie médicale 3D, de manière préopératoire, comme cela sera détaillé ci-après.

La présente invention concerne plus particulièrement un procédé de fabrication d'un dispositif d'aide à la pose d'une prothèse trapézo-métacarpienne tel que décrit ci-dessus, comprenant les étapes suivantes :
a) réaliser par imagerie médicale un modèle virtuel en trois dimensions (3D) au moins du trapèze 4 et du premier métacarpien 6 d'un patient, de manière préopératoire
b) déterminer sur les modèles virtuels 3D obtenus à l'étape a) le meilleur positionnement et la meilleure orientation possibles pour la mise en place d'un outil dans le trapèze 4 par rapport à l'anatomie du trapèze 4 et du premier métacarpien 6 du patient en vue de la pose de la cupule dans le trapèze 4 et de la tige dans le premier métacarpien 6
c) déterminer sur les modèles virtuels 3D obtenus à l'étape a) des lignes de résection 22 du premier métacarpien 6
d) obtenir sur les modèles virtuels 3D obtenus à l'étape a) la surface articulaire 4a du trapèze 4 et la surface articulaire 6a du premier métacarpien 6
e) créer une image 3D des premier et second guides 2, 28, la première surface transversale 8a du premier corps 8 du premier guide 2 étant configurée de manière à être congruente à la surface articulaire 4a du trapèze 4 déterminée à l'étape d), et la deuxième surface transversale 8b du premier corps 8 du premier guide 2 ainsi que la troisième surface transversale 30a du second corps 30 du second guide 28 étant configurées de manière à être congruentes l'une avec l'autre, et de préférence de manière à être toutes les deux congruentes à la surface articulaire 6a du premier métacarpien 6 déterminée à l'étape d)
f) déterminer la position des organes de repérage 20 sur l'image 3D du premier guide 2 créée à l'étape e) en fonction des lignes de résection 22 déterminées à l'étape c)
g) déterminer la position des premiers et seconds organes de fixation 10, 36 et des organes de positionnement 14, 16 sur les images 3D des premier et second guides 2, 28 créées à l'étape e)
h) déterminer la position (point d'entrée) et l'angle d'inclinaison α des premier et second orifices de guidage 24, 32 sur les images 3D respectivement des premier et second guides 2, 28 créées à l'étape e) en fonction du meilleur positionnement et de la meilleure orientation déterminées pour la mise en place d'un outil à l'étape b)
i) réaliser les premier et second guides 2, 28 selon les images 3D créées aux étapes e) à h).

L'imagerie médicale utilisée dans la présente invention peut être mise en oeuvre au moyen d'un scanner CT par exemple, ou tout autre moyen approprié. Les modèles virtuels du trapèze et du premier métacarpien sont reconstruits en 3D à partir des images CT-scan préopératoires du patient en utilisant des logiciels de modélisation numérique, tel qu'un logiciel de conception assistée par ordinateur (CAO) ou tout autre logiciel de traitement d'image disponible sur le marché.

Les premier et second guides 2 et 28 sont conçus à partir des modèles virtuels 3D du patient selon le procédé décrit ci-dessus et sont fabriqués de préférence selon l'étape i) par impression 3D. Il est bien évident que tout autre procédé d'usinage connu peut également être utilisé. Les premier et second guides 2 et 28 sont réalisés en matériaux compatibles avec les imprimantes 3D ou avec d'autres procédés d'usinage, ainsi qu'avec les exigences de stérilisation. De préférence, les matériaux pour réaliser les premier et second guides 2 et 28 peuvent être du polyétheréthercétone (PEEK), du polypropylène, du polyuréthane, du polyphénylsulfone (PPSU), des alliages à base de titane, des inox tel que l'inox 14021, ou tout autre matériau approprié. Puis les premier et second guides 2 et 28 sont stérilisés pour pouvoir ensuite être utilisés par le chirurgien pendant l'intervention.

Lors de l'intervention, après incision sur l'articulation trapézo-métacarpienne et exposition du premier métacarpien 6, le premier guide 2 est interposé entre le trapèze 4 et le premier métacarpien 6 puis fixé temporairement au trapèze par les pattes de fixation 10. Grâce aux surfaces 8a et 8b du premier guide 2 congruentes avec respectivement la surface articulaire 4a du trapèze et la surface articulaire 6a du premier métacarpien 6, aidées éventuellement par les pattes de centrage 14 et les pattes de positionnement 16, configurées de manière spécifique au patient, le premier guide 2 est interposé de manière stable, précise, et spécifique au patient, entre la surface articulaire 4a du trapèze 4 et la surface articulaire 6a du premier métacarpien.

Puis la base du premier métacarpien 6 est réséquée en suivant les lignes de résection 22 indiquées par les pattes de découpe 20 ou autre organe de repérage. La partie du premier métacarpien réséquée est éliminée, ce qui permet de pouvoir écarter légèrement le premier métacarpien du trapèze. Le canal médullaire pourra être ensuite préparé pour recevoir la tige d'une manière connue en soi.

Le second guide 28 est ensuite interposé entre le premier guide 2 et le premier métacarpien 6 réséqué, puis fixé temporairement au premier guide par les ergots de fixation 36. Grâce à la surface 30a du second guide 28 congruente avec la deuxième surface transversale 8b du premier guide 2, aidée éventuellement par les ergots de fixation 36, configurés de manière spécifique au patient, le second guide 28 est interposé de manière stable, précise, et spécifique au patient, entre le premier guide 2 et le premier métacarpien réséqué. Le premier guide 2 et le second guide 28 sont assemblés de sorte que le premier orifice de guidage traversant 24 et le second orifice de guidage traversant 32 sont alignés et communicants de manière à constituer un chemin de guidage suffisamment long et précis pour assurer et maintenir un positionnement et une orientation optimaux, spécifiques au patient, d'une broche de guidage 34 placée dans lesdits premier et second orifices de guidage 24 et 32. Après mise en place de la broche de guidage 34 dans lesdits premier et second orifices de guidage 24 et 32, ladite broche de guidage 34 est introduite dans le trapèze 4 au moyen d'un moteur. Les premier et second guides 2 et 28 sont ensuite retirés. Puis une fraise canulée est introduite sur la broche de guidage correctement positionnée et orientée dans le trapèze 4. Le chirurgien peut ensuite procéder au fraisage du trapèze pour la mise en place de la cupule de manière traditionnelle. La broche de guidage 34 restée en place dans le trapèze 4 présente le meilleur positionnement et la meilleure orientation possibles définis en fonction de l'anatomie avant opération du patient pour le positionnement de la prothèse trapézo-métacarpienne.

Le dispositif d'aide à la pose d'une prothèse trapézo-métacarpienne fabriqué selon le procédé selon l'invention permet de proposer à la fois un guide de coupe du premier métacarpien ainsi qu'un guide de positionnement et d'orientation d'une broche de guidage configurés de manière optimale spécifiquement pour le patient, nécessitant peu de place, ce qui lui permet de pouvoir être interposé dans l'espace réduit de l'articulation trapézo-métacarpienne. Le dispositif d'aide à la pose d'une prothèse trapézo-métacarpienne selon l'invention peut être positionné d'une manière très précise et spécifique au patient sur l'articulation trapézo-métacarpienne. Il reste en place pendant toute la partie de l'intervention jusqu'à la mise en place de la broche de guidage correctement placée et orientée, de sorte que tous les repères de positionnement de la prothèse trapézo-métacarpienne définis selon l'anatomie du patient avant opération sont préservés. Le dispositif d'aide à la pose selon la présente invention permet de positionner une prothèse trapézo-métacarpienne d'une manière optimale, respectueuse de l'anatomie du patient.

## Revendications

1. Procédé de fabrication d'un dispositif (1) d'aide à la pose d'une prothèse trapézo-métacarpienne comprenant une cupule destinée à être fixée dans le trapèze (4) d'un patient et une tige destinée à être fixée dans le premier métacarpien (6) du patient, ledit dispositif (1) étant réalisé de telle sorte qu'il comprend un premier guide (2) apte à être interposé entre le trapèze (4) et le premier métacarpien (6) du patient, ledit premier guide (2) comprenant un premier corps (8) présentant une première surface (8a) destinée à être positionnée sur la surface articulaire (4a) du trapèze (4), ladite première surface (8a) étant congruente à ladite surface articulaire (4a) du trapèze (4), et une deuxième surface (8b) destinée à être en regard de la surface articulaire (6a) du premier métacarpien (6) avant résection, et des organes de repérage (20) portés par ledit premier corps (8) et agencés pour définir des lignes de résection (22) du premier métacarpien (6), le premier guide (2) étant réalisé au moins partiellement sur mesure à l'aide d'une imagerie médicale en trois dimensions (3D) au moins du trapèze (4) et du premier métacarpien (6) du patient, de manière préopératoire.

2. Procédé de fabrication selon la revendication 1, ledit dispositif (1) étant réalisé de telle sorte que la deuxième surface (8b) du premier corps (8) du premier guide (2) est congruente à la surface articulaire (6a) du premier métacarpien (6) afin d'être apte à être positionnée sur ladite surface articulaire (6a) du premier métacarpien (6) avant résection.

3. Procédé de fabrication selon l'une des revendications précédentes, ledit dispositif (1) étant réalisé de telle sorte que le premier guide (2) comprend des premiers organes de fixation (10) agencés pour renforcer le maintien du premier corps (8) du premier guide (2) au trapèze (4).

4. Procédé de fabrication selon l'une des revendications précédentes, ledit dispositif (1) étant réalisé de telle sorte que le premier guide (2) comprend des organes de positionnement (14, 16) agencés pour garantir l'interposition du premier corps (8) du premier guide (2) entre le trapèze (4) et le premier métacarpien (6).

5. Procédé de fabrication selon l'une des revendications précédentes, ledit dispositif (1) étant réalisé de telle sorte que le premier corps (8) du premier guide (2) comprend au moins sur sa deuxième surface (8b) au moins un premier orifice de guidage (24) agencé pour recevoir un outil (34).

6. Procédé de fabrication selon l'une des revendications précédentes, ledit dispositif (1) étant réalisé de telle sorte qu'il comprend un second guide (28) agencé pour être positionné sur le premier guide (2) après résection du premier métacarpien (6), ledit second guide (28) comprenant un second corps (30) présentant une troisième surface (30a) destinée à reposer sur la deuxième surface (8b) du premier guide (2), et une quatrième surface (30b) destinée à être en regard du premier métacarpien (6) réséqué, ladite troisième surface (30a) étant congruente avec la deuxième surface (8b) du premier guide (2), et de telle sorte que ledit second corps (30) du second guide (28) comprend au moins sur sa quatrième surface (30b) au moins un second orifice de guidage (32) agencé pour recevoir un outil (34).

7. Procédé de fabrication selon les revendications 5 et 6, ledit dispositif (1) étant réalisé de telle sorte que les premier et second orifices de guidage (24, 32) sont alignés, traversants et communicants.

8. Procédé de fabrication selon l'une des revendications 6 et 7, ledit dispositif (1) étant réalisé de telle sorte que le second guide (28) comprend des seconds organes de fixation (36) agencés pour maintenir le second corps (30) du second guide (28) sur le premier guide (2).

9. Procédé de fabrication selon l'une des revendications précédentes, la position et l'inclinaison du premier orifice de guidage (24) du premier guide (2) étant définies de manière préopératoire afin de permettre la mise en place d'un outil (34) dans le premier guide (2) selon le meilleur positionnement et la meilleure orientation possibles par rapport à l'anatomie du trapèze (4) et du premier métacarpien (6) du patient.

10. Procédé de fabrication selon l'une des revendications 6 à 9, le second guide (28) étant réalisé au moins partiellement sur mesure à l'aide d'une imagerie médicale en trois dimensions (3D) au moins du trapèze (4) et du premier métacarpien (6) du patient, de manière préopératoire.

11. Procédé de fabrication selon la revendication 10, la position et l'inclinaison du second orifice de guidage (32) du second guide (28) étant définies de manière préopératoire afin de permettre la mise en place d'un outil (34) dans le second guide (28) selon le meilleur positionnement et la meilleure orientation possibles par rapport à l'anatomie du trapèze (4) et du premier métacarpien (6) du patient.

12. Procédé de fabrication selon les revendications 1 à 8, comprenant les étapes suivantes :
a) réaliser par imagerie médicale un modèle virtuel en trois dimensions (3D) au moins du trapèze (4) et du premier métacarpien (6) du patient, de manière préopératoire
b) déterminer sur les modèles virtuels 3D obtenus à l'étape a) le meilleur positionnement et la meilleure orientation possibles pour la mise en place d'un outil (34) dans le trapèze (4) par rapport à l'anatomie du trapèze (4) et du premier métacarpien (6) du patient
c) déterminer sur les modèles virtuels 3D obtenus à l'étape a) des lignes de résection (22) du premier métacarpien (6)
d) obtenir sur les modèles virtuels 3D obtenus à l'étape a) la surface articulaire (4a) du trapèze (4) et la surface articulaire (6a) du premier métacarpien (6)
e) créer une image 3D des premier et second guides (2, 28), la première surface (8a) du premier guide (2) étant configurée de manière à être congruente à la surface articulaire (4a) du trapèze (4) déterminée à l'étape d), et la deuxième surface (8b) du premier guide (2) ainsi que la troisième surface (30a) du second guide (28) étant configurées de manière à être congruentes l'une avec l'autre
f) déterminer la position des organes de repérage (20) sur l'image 3D du premier guide (2) créée à l'étape e) en fonction des lignes de résection (22) déterminées à l'étape c)
g) déterminer la position des premiers et seconds organes de fixation (10, 36) et des organes de positionnement (14, 16) sur les images 3D des premier et second guides (2, 28) créées à l'étape e)
h) déterminer la position des premier et second orifices de guidage (24, 32) sur les images 3D respectivement des premier et second guides (2, 28) créées à l'étape e) en fonction du meilleur positionnement et de la meilleure orientation déterminées pour la mise en place d'un outil (34) à l'étape b)
i) réaliser les premier et second guides (2, 28) selon les images 3D créées aux étapes e) à h).

13. Procédé de fabrication selon la revendication 12, la deuxième surface (8b) du premier guide (2) configurée à l'étape e) étant configurée de manière à être congruente à la surface articulaire (6a) du premier métacarpien (6) déterminée à l'étape d).

## Patentansprüche

1. Verfahren zum Herstellen einer Hilfsvorrichtung (1) zum Einsetzen einer Daumensattelgelenkprothese, umfassend eine Schale zum Befestigen im großen Vieleckbein (4) eines Patienten und einen Stab zum Befestigen im ersten Mittelhandknochen (6) des Patienten, wobei die Vorrichtung (1) derart ausgebildet ist, dass sie eine erste Führung (2) umfasst, die dazu geeignet ist, zwischen dem großen Vieleckbein (4) und dem ersten Mittelhandknochen (6) des Patienten eingeschoben zu werden, wobei die erste Führung (2) einen ersten Körper (8) umfasst, der eine erste Oberfläche (8a) zum Positionieren auf der Gelenkfläche (4a) des großen Vieleckbeins (4), wobei die erste Oberfläche (8a) kongruent mit der Gelenkfläche (4a) des großen Vieleckbeins (4) ist, und eine zweite Oberfläche (8b), die dazu bestimmt ist, sich vor einer Resektion gegenüber der Gelenkfläche (6a) des ersten Mittelhandknochens (6) zu befinden, aufweist, und Markierungsglieder (20), die von dem ersten Körper (8) getragen werden und eingerichtet sind, um die Resektionslinien (22) des ersten Mittelhandknochens (6) zu definieren, wobei die erste Führung (2) anhand einer medizinischen Bildgebung in drei Dimensionen (3D) mindestens des großen Vieleckbeins (4) und des ersten Mittelhandknochens (6) des Patienten präoperativ mindestens teilweise maßgefertigt wird.

2. Herstellungsverfahren nach Anspruch 1, wobei die Vorrichtung (1) derart ausgebildet ist, dass die zweite Oberfläche (8b) des ersten Körpers (8) der ersten Führung (2) mit der Gelenkfläche (6a) des ersten Mittelhandknochens (6) kongruent ist, um vor der Resektion auf der Gelenkfläche (6a) des ersten Mittelhandknochens (6) positioniert werden zu können.

3. Herstellungsverfahren nach einem der vorhergehenden Ansprüche, wobei die Vorrichtung (1) derart ausgebildet ist, dass die erste Führung (2) erste Befestigungsglieder (10) umfasst, die dazu eingerichtet sind, den Halt des ersten Körpers (8) der ersten Führung (2) an dem großen Vieleckbein (4) zu verstärken.

4. Herstellungsverfahren nach einem der vorhergehenden Ansprüche, wobei die Vorrichtung (1) derart ausgebildet ist, dass die erste Führung (2) Positionierungsglieder (14, 16) umfasst, die dazu eingerichtet sind, das Einschieben des ersten Körpers (8) der ersten Führung (2) zwischen dem großen Vieleckbein (4) und dem ersten Mittelhandknochen (6) zu gewährleisten.

5. Herstellungsverfahren nach einem der vorhergehenden Ansprüche, wobei die Vorrichtung (1) derart ausgebildet ist, dass der erste Körper (8) der ersten Führung (2) mindestens auf seiner zweiten Oberfläche (8b) mindestens eine erste Führungsöffnung (24) umfasst, die dazu eingerichtet ist, ein Instrument (34) aufzunehmen.

6. Herstellungsverfahren nach einem der vorhergehenden Ansprüche, wobei die Vorrichtung (1) derart ausgebildet ist, dass sie eine zweite Führung (28) umfasst, die dazu eingerichtet ist, nach der Resektion des ersten Mittelhandknochens (6) auf der ersten Führung (2) positioniert zu werden, wobei die zweite Führung (28) einen zweiten Körper (30) umfasst, der eine dritte Oberfläche (30a), die dazu bestimmt ist, auf der zweiten Oberfläche (8b) der ersten Führung (2) zu liegen, und eine vierte Oberfläche (30b), die dazu bestimmt ist, dem resezierten ersten Mittelhandknochen (6) gegenüberzuliegen, aufweist, wobei die dritte Oberfläche (30a) mit der zweiten Oberfläche (8b) der ersten Führung (2) kongruent ist, und dass der zweite Körper (30) der zweiten Führung (28) mindestens auf seiner vierten Oberfläche (30b) mindestens eine zweite Führungsöffnung (32) umfasst, die dazu eingerichtet ist, ein Instrument (34) aufzunehmen.

7. Herstellungsverfahren nach Anspruch 5 und 6, wobei die Vorrichtung (1) derart ausgebildet ist, dass die erste und die zweite Führungsöffnung (24, 32) ausgerichtet, durchquerend und kommunizierend sind.

8. Herstellungsverfahren nach einem der Ansprüche 6 und 7, wobei die Vorrichtung (1) derart ausgebildet ist, dass die zweite Führung (28) zweite Befestigungsglieder (36) umfasst, die dazu eingerichtet sind, den zweiten Körper (30) der zweiten Führung (28) auf der ersten Führung (2) zu halten.

9. Herstellungsverfahren nach einem der vorhergehenden Ansprüche, wobei die Position und Neigung der ersten Führungsöffnung (24) der ersten Führung (2) präoperativ definiert werden, um das Einsetzen eines Instruments (34) in die erste Führung (2) gemäß der bestmöglichen Positionierung und der bestmöglichen Orientierung im Verhältnis zur Anatomie des großen Vieleckbeins (4) und des ersten Mittelhandknochens (6) des Patienten zu ermöglichen.

10. Herstellungsverfahren nach einem der Ansprüche 6 bis 9, wobei die zweite Führung (28) anhand einer medizinischen Bildgebung in drei Dimensionen (3D) mindestens des großen Vieleckbeins (4) und des ersten Mittelhandknochens (6) des Patienten präoperativ mindestens teilweise maßgefertigt wird.

11. Herstellungsverfahren nach Anspruch 10, wobei die Position und die Neigung der zweiten Führungsöffnung (32) der zweiten Führung (28) präoperativ definiert werden, um das Einsetzen eines Instruments (34) in die zweite Führung (28) gemäß der bestmöglichen Positionierung und der bestmöglichen Orientierung im Verhältnis zur Anatomie des großen Vieleckbeins (4) und des ersten Mittelhandknochens (6) des Patienten zu ermöglichen.

12. Herstellungsverfahren nach Anspruch 1 bis 8, umfassend folgende Schritte:
a) präoperatives Ausbilden durch medizinische Bildgebung eines virtuellen Modells in drei Dimensionen (3D) mindestens des großen Vieleckbeins (4) des ersten Mittelhandknochens (6) des Patienten,
b) Bestimmen an den in Schritt a) erzielten virtuellen 3D-Modellen der bestmöglichen Positionierung und der bestmöglichen Orientierung für das Einsetzen eines Instruments (34) in das große Vieleckbein (4) im Verhältnis zur Anatomie des großen Vieleckbeins (4) und des ersten Mittelhandknochens (6) des Patienten,
c) Bestimmen an den in Schritt a) erzielten virtuellen 3D-Modellen der Resektionslinien (22) des ersten Mittelhandknochens (6),
d) Erzielen an den in Schritt a) erzielten virtuellen 3D-Modellen der Gelenkfläche (4a) des großen Vieleckbeins (4) und der Gelenkfläche (6a) des ersten Mittelhandknochens (6),
e) Erstellen eines 3D-Bildes der ersten und der zweiten Führung (2, 28), wobei die erste Oberfläche (8a) der ersten Führung (2) derart konfiguriert ist, dass sie mit der in Schritt d) bestimmten Gelenkfläche (4a) des großen Vieleckbeins (4) kongruent ist, und die zweite Oberfläche (8b) der ersten Führung (2) sowie die dritte Oberfläche (30a) der zweiten Führung (28) derart konfiguriert sind, dass sie miteinander kongruent sind,
f) Bestimmen der Position der Markierungsglieder (20) in dem 3D-Bild der ersten Führung (2), das in Schritt e) erstellt wird, in Abhängigkeit von den in Schritt c) bestimmten Resektionslinien (22),
g) Bestimmen der Position der ersten und zweiten Befestigungsglieder (10, 36) und der Positionierungsglieder (14, 16) in den in Schritt e) erstellten 3D-Bildern der ersten und der zweiten Führung (2, 28),
h) Bestimmen der Position der ersten und der zweiten Führungsöffnung (24, 32) in den in Schritt e) erstellten 3D-Bildern jeweils der ersten und der zweiten Führung (2, 28) in Abhängigkeit von der besten Positionierung und der besten Orientierung, die für das Einsetzen eines Instruments (34) in Schritt b) bestimmt werden,
i) Ausbilden der ersten und der zweiten Führung (2, 28) gemäß den in Schritt e) bis h) erstellten 3D-Bildern.

13. Herstellungsverfahren nach Anspruch 12, wobei die zweite Oberfläche (8b) der ersten Führung (2), die in Schritt e) konfiguriert wird, derart konfiguriert wird, dass sie mit der Gelenkfläche (6a) des ersten Mittelhandknochens (6), die in Schritt d) bestimmt wird, kongruent ist.

## Claims

1. Method of producing a device (1) for assisting in the placement of a trapeziometacarpal prosthesis comprising a cup intended to be fixed in the trapezium (4) of a patient and a stem intended to be fixed in the patient's first metacarpal (6), wherein said device (1) is produced such that it comprises a first guide (2) able to be interposed between the trapezium (4) and the first metacarpal (6) of the patient, said first guide (2) comprising a first body (8) having a first surface (8a) intended to be positioned on the articular surface (4a) of the trapezium (4), said first surface (8a) being congruent with said articular surface (4a) of the trapezium (4), and a second surface (8b) intended to face the articular surface (6a) of the first metacarpal (6) before resection, and locating members (20) carried by said first body (8) and arranged to define resection lines (22) for the first metacarpal (6), and wherein the first guide (2) is produced at least partially on an individualised basis with the aid of medical three-dimensional (3D) imaging at least of the trapezium (4) and of the first metacarpal (6) of the patient, pre-operatively.

2. Producing method as claimed in claim 1, wherein said device (1) is produced such that the second surface (8b) of the first body (8) of the first guide (2) is congruent with the articular surface (6a) of the first metacarpal (6) so as to be suitable to be positioned on said articular surface (6a) of the first metacarpal (6) before resection.

3. Producing method as claimed in any one of the preceding claims, wherein said device (1) is produced such that the first guide (2) comprises first fixing members (10) arranged to reinforce the holding of the first body (8) of the first guide (2) on the trapezium (4).

4. Producing method as claimed in any one of the preceding claims, wherein said device (1) is produced such that the first guide (2) comprises positioning members (14, 16) arranged to guarantee the interpositioning of the first body (8) of the first guide (2) between the trapezium (4) and the first metacarpal (6).

5. Producing method as claimed in any one of the preceding claims, wherein said device (1) is produced such that the first body (8) of the first guide (2) comprises, at least on its second surface (8b), at least one first guide orifice (24) arranged to receive a tool (34).

6. Producing method as claimed in any one of the preceding claims, wherein said device (1) is produced such that it comprises a second guide (28) arranged to be positioned on the first guide (2) after resection of the first metacarpal (6), said second guide (28) comprising a second body (30) having a third surface (30a) intended to rest on the second surface (8b) of the first guide (2), and a fourth surface (30b) intended to face the resected first metacarpal (6), said third surface (30a) being congruent with the second surface (8b) of the first guide (2), and such that that said second body (30) of the second guide (28) comprises, at least on its fourth surface (30b), at least one second guide orifice (32) arranged to receive a tool (34).

7. Producing method as claimed in claims 5 and 6, wherein said device (1) is produced such that the first and second guide orifices (24, 32) are aligned, through-going and communicating.

8. Producing method as claimed in any one of claims 6 and 7, wherein said device (1) is produced such that the second guide (28) comprises second fixing members (36) arranged to hold the second body (30) of the second guide (28) on the first guide (2).

9. Producing method as claimed in any one of the preceding claims, wherein the position and the inclination of the first guide orifice (24) of the first guide (2) are defined pre-operatively in order to permit the placement of a tool (34) in the first guide (2) according to the best positioning and the best orientation possible with respect to the anatomy of the trapezium (4) and of the first metacarpal (6) of the patient.

10. Producing method as claimed in any one of claims 6 to 9, wherein the second guide (28) is produced at least partially on an individualised basis with the aid of medical three-dimensional (3D) imaging at least of the trapezium (4) and of the first metacarpal (6) of the patient, pre-operatively.

11. Producing method as claimed in claim 10, wherein the position and the inclination of the second guide orifice (32) of the second guide (28) are defined pre-operatively in order to permit the placement of a tool (34) in the second guide (28) according to the best positioning and the best orientation possible with respect to the anatomy of the trapezium (4) and of the first metacarpal (6) of the patient.

12. Producing method as claimed in claims 1 to 8, comprising the following steps:
a) producing, by medical imaging, a virtual model in three dimensions (3D) at least of the trapezium (4) and of the first metacarpal (6) of the patient, pre-operatively
b) determining on the virtual 3D models obtained in step a) the best positioning and the best orientation possible for the placement of a tool (34) in the trapezium (4) with respect to the anatomy of the trapezium (4) and of the first metacarpal (6) of the patient
c) determining, on the virtual 3D models obtained in step a), resection lines (22) for the first metacarpal (6)
d) obtaining on the virtual 3D models obtained in step a) the articular surface (4a) of the trapezium (4) and the articular surface (6a) of the first metacarpal (6)
e) creating a 3D image of the first and second guides (2, 28), the first surface (8a) of the first guide (2) being configured to be congruent to the articular surface (4a) of the trapezium (4) determined in step d), and the second surface (8b) of the first guide (2) as well as the third surface (30a) of the second guide (28) being configured to be congruent with each other
f) determining the position of the locating members (20) on the 3D image of the first guide (2) created in step e) according to the resection lines (22) determined in step c)
g) determining the position of the first and second fixing members (10, 36) and of the positioning members (14, 16) on the 3D images of the first and second guides (2, 28) created in step e)
h) determining the position of the first and second guide orifices (24, 32) on the 3D images respectively of the first and second guides (2, 28) created in step e) according to the best positioning and the best orientation determined for the placement of a tool (34) in step b)
i) producing the first and second guides (2, 28) according to the 3D images created in steps e) to h).

13. Producing method of producing as claimed in claim 12, wherein the second surface (8b) of the first guide (2) configured in step e) is configured to be congruent with the articular surface (6a) of the first metacarpal (6) determined in step d).
